# DEMANDE DE BREVET EUROPEEN

(11) **EP 1 961 456 A1**
(43) Date de publication de la demande: **27.08.2008**
(21) Numéro de dépôt: 08151432.5
(22) Date de dépôt: 14.02.2008
(51) Int. Cl.: A61Q 19/10, A61K 8/02, A61Q 1/14

(54) **Lingette imprégnée d'une émulsion épaisse**

(30) Priorité: 21.02.2007 FR 0753397
(71) Demandeur: L'Oreal, 75008 Paris (FR)
(72) Inventeur: Potin, Anthony, 75006, PARIS (FR)
(74) Mandataire: Duvert, Sandra

(57) **Abrégé**

L'invention se rapporte à un article cosmétique comportant un substrat insoluble dans l'eau et une composition ajoutée ou imprégnée sur le substrat, comprenant au moins une phase aqueuse, au moins une phase huileuse, et un ou plusieurs polymères semi-cristallins.

Cet article peut constituer notamment une lingette qui peut être utilisée en particulier pour le soin de la peau et/ou des cheveux, et/ou pour le nettoyage et/ou le démaquillage de la peau.

## Description

L'invention se rapporte à un article cosmétique, plus particulièrement une lingette, comportant au moins un substrat insoluble dans l'eau et une composition contenant au moins un polymère semi-cristallin, ainsi qu'aux utilisations dudit article dans le domaine cosmétique ou dermatologique, en particulier pour le soin et le traitement de la peau humaine, le nettoyage et/ou le démaquillage de la peau humaine, plus spécialement du visage, et le soin des cheveux.

Les lingettes cosmétiques sont généralement constituées d'un substrat en une matière d'origine naturelle ou synthétique, qui est de préférence un non tissé, mais qui peut être aussi une mousse ou un tissu, ledit substrat étant imprégné d'une composition adaptée au but recherché, par exemple le nettoyage ou le démaquillage de la peau ou des yeux, ou encore le soin de la peau ou des cheveux. De telles lingettes sont couramment utilisées, car elles sont appréciées pour leur coté pratique du fait qu'elles sont jetables et qu'elles sont imprégnées de la quantité nécessaire et suffisante de produit traitant ou nettoyant. L'utilisation de ces lingettes évite la manipulation et le transport de flacons contenant les compositions cosmétiques.

L'imprégnation des substrats par la composition d'imprégnation peut se faire selon différentes techniques telles que la pulvérisation ou le trempage. Toutefois, ces techniques ne peuvent être utilisées que si les compositions d'imprégnation sont suffisamment fluides et ont une viscosité proche de celle de l'eau. En effet, il n'est pas possible de mouiller correctement le substrat quand les compositions sont trop visqueuses, car, alors, le substrat est incorrectement imprégné et, de plus, il est difficile à découper, plier et ensacher. En outre, l'article obtenu est désagréable à utiliser car le produit d'imprégnation reste en surface du substrat ou ne l'imprègne pas de manière homogène, si bien que certaines zones de l'article contiennent trop de produit, alors que d'autres en sont dépourvues ou en contiennent trop peu.

Par ailleurs, les compositions fluides imprégnant les lingettes présentent l'inconvénient de contenir peu d'huile et, de ce fait, d'avoir un effet de soin de la peau ou un pouvoir démaquillant, jugés insuffisants. De plus, les lingettes obtenues présentent un manque de confort à l'application.

Il existe des lingettes comprenant une composition à base d'huiles, qui sont utilisées pour le soin de la peau (imprégnation d'huiles pour le corps) ou pour le démaquillage de la peau (imprégnation d'huiles démaquillantes), mais lors de l'application sur la peau, ces lingettes laissent un film gras que la lingette, elle-même imbibée d'huile, ne peut pas éliminer. Ceci est particulièrement désagréable au niveau de l'oeil quand la lingette est utilisée pour le démaquillage des yeux. Par ailleurs, des lingettes contenant des huiles associées à des tensioactifs qui sont utilisées après humidification avec un peu d'eau, l'addition d'eau permettant d'obtenir une émulsion par dispersion du mélange d'huile et de tensioactif dans l'eau, mais l'émulsion ainsi obtenue reste très liquide, sans consistance et elle a tendance à couler hors du substrat, ce qui est désagréable pour l'utilisateur.

Ainsi, les compositions d'imprégnation des lingettes utilisées jusqu'à présent soit sont trop grasses soit manquent de consistance et ne permettent pas d'obtenir une texture crémeuse, critères importants pour atteindre un bon confort d'utilisation aussi bien dans le soin que dans le démaquillage de la peau. Or, le confort apporté par un produit cosmétique, lors de son utilisation et immédiatement après son utilisation, est tout aussi important que son efficacité. Le fait d'obtenir une crème et non un fluide apporte un confort sur la peau, très appréciable pour l'utilisateur. De plus, le fait d'avoir une composition plus épaisse et plus riche en huiles permet aussi d'améliorer l'efficacité du produit pour le soin et/ou le démaquillage de la peau.

Ainsi, il subsiste le besoin de disposer d'un article (lingette ou compresse ou mousse) pouvant donner lors de l'application sur la peau, une composition onctueuse comme une crème, cet article étant à la fois facile à utiliser, confortable à l'application et efficace.

La demanderesse a trouvé de manière surprenante qu'une composition sous forme d'émulsion, contenant un ou plusieurs polymères semi-cristallins, pouvait être imprégnée de manière homogène par les moyens conventionnels sur une lingette ou tout autre substrat insoluble dans l'eau. La composition selon l'invention présente l'avantage de donner une imprégnation bien homogène sur la lingette, ce qui n'est pas le cas avec d'autres épaississants de phase huileuse.

La présente invention a donc pour objet un article cosmétique comportant (A) un substrat insoluble dans l'eau, comprenant une ou plusieurs couches, et (B) une composition ajoutée ou imprégnée sur le substrat, comprenant au moins une phase aqueuse, au moins une phase huileuse, et un ou plusieurs polymères semi-cristallins.

On entend ici par « article cosmétique », l'ensemble constitué par un support insoluble dans l'eau et une composition cosmétique imprégnée sur le support. Cet article peut être en particulier une lingette mais il peut aussi avoir toute forme appropriée comme décrit ci-après. Le substrat insoluble dans l'eau doit être absorbant et suffisamment résistant pour ne pas se déliter lors de son utilisation.

La composition de l'invention a la consistance d'une crème, et elle présente généralement une viscosité allant de préférence de 1 Pa.s à 15 Pa.s, de préférence 1 à 10 Pa.s et mieux de 1,5 Pa.s à 10 Pa.s, cette viscosité étant mesurée à la température ambiante (environ 25°C) avec un appareil RHEOMAT RM 180, mobiles 1 à 4 selon la viscosité de la composition.

L'article selon l'invention est humide au toucher. Il présente l'avantage d'être confortable lors de l'application sur la peau, et d'avoir un effet nourrissant du fait de la présence d'une phase huileuse. De plus, l'imprégnation est bien homogène grâce à la présence des polymères semi-cristallins.

En outre, cet article est très facile à manipuler lors de l'utilisation. Quand c'est un article de soin de la peau, il est appliqué sur la peau de manière à rester collé sur la peau, sur laquelle il doit rester un temps suffisant pour que la composition imprègne la peau, ce temps pouvant aller par exemple de 5 à 30 minutes. Puis, on enlève l'article, et on essuie l'excédent de composition présent sur la peau. Cet article peut constituer aussi un masque de soin. On peut l'utiliser de la même manière sur les cheveux.

Quand il est utilisé pour le nettoyage ou le démaquillage de la peau, on le passe sur la peau en le laissant éventuellement appliqué un temps suffisant pour que les produits de maquillage se dissolvent dans la composition d'imprégnation de l'article, et puis on essuie la peau. On peut aussi éventuellement rincer ensuite la peau.

L'article selon l'invention est en particulier un article cosmétique, approprié pour le soin et/ou le traitement de la peau du visage, du corps ou des mains, et pour le nettoyage ou le démaquillage de la peau du visage et/ou du corps. Il peut être utilisé aussi pour le soin des cheveux, ainsi que pour le démaquillage des yeux.

L'article selon l'invention peut avoir toute forme appropriée au but recherché. Il peut constituer une lingette, mais il peut aussi se trouver sous forme d'un gant, d'une moufle ou sous toute autre forme appropriée pour une utilisation pratique sur le visage ou le corps, par exemple sous forme d'un visage avec les trous pour les emplacements des yeux, du nez et/ou de la bouche, ou sous forme d'un doigtier démaquillant pour une application pour le démaquillage des cils, ou sous forme d'un disque simple ou double face pouvant contenir notamment deux faces imprégnées de compositions différentes. La forme de gant convient particulièrement pour le traitement des mains sèches ou gercées, car la main peut être laissée dans le gant, pendant un temps suffisant pour que la composition imprègne la peau des mains et lui apporte le bienfait des huiles qu'elle contient. L'article peut aussi comporter une surface rugueuse permettant l'exfoliation (gommage) de la peau.

L'invention a aussi pour objet l'utilisation cosmétique de l'article tel que défini ci-dessus, pour le soin de la peau ou des cheveux, et/ou le nettoyage et/ou le démaquillage et/ou le gommage de la peau.

La composition utilisée selon l'invention pour l'imprégnation du substrat insoluble dans l'eau, étant destinée à une application topique, contient un milieu physiologiquement acceptable, c'est-à-dire compatible avec la peau, les muqueuses, les cheveux et le cuir chevelu.

La composition de l'invention comprend au moins une phase aqueuse et au moins une phase huileuse, et elle peut se trouver sous forme d'émulsion eau-dans-huile (E/H) ou d'émulsion huile-sans-eau (H/E) ou d'émulsion multiple (E/H/E ou H/E/H). De manière préférée, la composition est sous forme d'émulsion E/H.

### Polymères semi-cristallins

La composition selon l'invention contient au moins un polymère semi-cristallin, de préférence dérivé d'acide acrylique ou méthacrylique. Ce polymère est généralement dans la phase huileuse. Par "polymère semi-cristallin", on entend au sens de l'invention, des polymères comportant une partie cristallisable, chaîne pendante ou séquence dans le squelette, et une partie amorphe dans le squelette, et présentant une température de changement de phase réversible du premier ordre, en particulier de fusion (transition solide-liquide). Lorsque la partie cristallisable est une séquence du squelette polymérique, cette séquence cristallisable est de nature chimique différente de celle des séquences amorphes ; le polymère semi-cristallin est dans ce cas un polymère séquencé par exemple du type dibloc, tribloc ou multibloc.

De façon avantageuse, le ou les polymères semi-cristallins de la composition de l'invention ont une masse moléculaire moyenne en nombre Mn supérieure ou égale à 2000, allant par exemple de 2000 à 800000, de préférence de 3000 à 500000, par exemple de 4000 à 150000, et mieux de 4000 à 99000.

Dans la composition selon l'invention, les polymères semi-cristallins sont avantageusement solubles dans la phase huileuse à au moins 1 % en poids, à une température supérieure à leur température de fusion. En dehors des chaînes ou séquences cristallisables, les séquences des polymères sont amorphes. Par "chaîne ou séquence cristallisable", on entend au sens de l'invention une chaîne ou séquence qui, si elle était seule, passerait de l'état amorphe à l'état cristallin, de façon réversible, selon qu'on est au-dessus ou en dessous de la température de fusion. Une chaîne au sens de l'invention est un groupement d'atomes, pendant ou latéral par rapport au squelette du polymère. Une séquence est un groupement d'atomes appartenant au squelette, groupement constituant un des motifs répétitifs du polymère.

De préférence, le squelette polymérique des polymères semi-cristallins est soluble dans la phase huileuse.

De façon préférée, les polymères semi-cristallins utilisés dans la composition de l'invention présentent une température de fusion (ou point de fusion), pF, inférieure à 70°C (25°C ≤pF< 70°C), cette température étant au moins égale à la température de la matière kératinique devant recevoir la composition selon l'invention, notamment la peau. La température de fusion peut être mesurée notamment par toute méthode connue et en particulier avec un calorimètre à balayage différentiel (D.S.C).

De préférence, les séquences ou chaînes cristallisables des polymères semi-cristallins représentent au moins 30 % du poids total du polymère et mieux au moins 40 % du poids total du polymère. Les polymères semi-cristallins à séquences cristallisables utilisés selon l'invention sont de préférence des polymères séquencés ou multiséquencés. Ils peuvent être obtenus par polymérisation de monomère à doubles liaisons réactives (ou éthyléniques) ou par polycondensation. Lorsque les polymères de l'invention sont des polymères à chaînes latérales cristallisables, ils sont avantageusement sous forme aléatoire ou statistique.

Les polymères semi-cristallins de l'invention sont généralement d'origine synthétique. Ils sont de préférence choisis parmi les polymères (homopolymères ou copolymères) portant au moins une chaîne latérale cristallisable, et les polymères (homopolymères ou copolymères) portant dans le squelette au moins une séquence cristallisable, comme ceux décrits dans le document US-A-5,156,911. La ou les chaînes latérales ou séquences cristallisables sont hydrophobes.

Selon un mode préféré de réalisation de l'invention, les polymères semi-cristallins sont choisis notamment parmi les homopolymères et copolymères résultant de la polymérisation d'au moins un monomère à chaîne(s) cristallisable(s), la chaîne cristallisable étant choisie parmi les chaînes alkyles comportant au moins 11 atomes de carbone et au plus 40 atomes de carbone et mieux au plus 24 atomes de carbone. Il s'agit notamment de chaînes alkyle comportant au moins 12 atomes de carbone, et de préférence, il s'agit de chaînes alkyle comportant de 14 à 24 atomes de carbone (C₁₄-C₂₄). Il peut s'agir de chaînes alkyle hydrocarbonées (atomes de carbone et d'hydrogène) ou chaînes alkyle fluorées ou perfluorées (atomes de carbone, atomes de fluor et éventuellement atomes d'hydrogène). Lorsqu'il s'agit de chaînes alkyle fluorées ou perfluorées, elles comportent au moins 11 atomes de carbone dont au moins 6 atomes de carbone sont fluorés. Les chaînes alkyle sont de préférence des chaînes hydrocarbonées.

Par "alkyle", on entend au sens de l'invention un groupement saturé (ne comportant pas d'insaturation).

Selon un mode particulier de réalisation de l'invention, le polymère semi-cristallin est choisi parmi les polymères issus d'au moins un monomère à chaîne cristallisable choisi parmi les (méth)acrylates d'alkyle en C₁₄-C₂₄, les (méth)acrylates de perfluoroalkyle en C₁₁-C₁₅, les N alkyl (méth)acrylamides en C₁₄ à C₂₄ avec ou sans atome de fluor, les esters vinyliques à chaînes alkyle ou perfluoroalkyle en C₁₄ à C₂₄, les éthers vinyliques à chaînes alkyle ou perfluoroalkyle en C₁₄ à C₂₄, les alpha-oléfines en C₁₄ à C₂₄, les para-alkyl styrènes avec un groupe alkyle en C₁₄ à C₂₄, et les copolymères de ces monomères obtenus par copolymérisation de ces monomères avec un monomère hydrophile, de préférence différent de l'acide méthacrylique, comme par exemple la N-vinylpyrrolidone, l'hydroxyéthylacrylate, l'hydroxyéthylméthacrylate, l'acide acrylique. De tels copolymères peuvent être par exemple les copolymères d'alkyl-C₁₄-C₂₄-acrylate, d'alkyl-C₁₄-C₂₄-méthacrylate, d'alkyl-C₁₄-C₂₄-acrylamide, d'alkyl-C₁₄-C₂₄-méthacrylamide, avec la N-vinylpyrrolidone, l'hydroxyéthylacrylate, l'hydroxyéthylméthacrylate, l'acide acrylique, ou leurs mélanges.

Les polymères semi-cristallins de la composition de l'invention peuvent être non réticulés ou réticulés en partie, du moment que le taux de réticulation ne gène pas leur dissolution ou dispersion dans la phase huileuse par chauffage au-dessus de leur température de fusion. Il peut s'agir alors d'une réticulation chimique, par réaction avec un monomère multifonctionnel lors de la polymérisation. Il peut aussi s'agir d'une réticulation physique qui peut alors être due soit à l'établissement de liaisons type hydrogène ou dipolaire entre des groupes portés par le polymère, comme par exemple les interactions dipolaires entre ionomères carboxylates, ces interactions étant en faible quantité et portées par le squelette du polymère ; soit à une séparation de phase entre les séquences cristallisables et les séquences amorphes portées par le polymère.

De préférence, les polymères semi-cristallins de la composition selon l'invention sont non réticulés.

Selon un mode particulier de réalisation de l'invention, le polymère semi-cristallin est choisi parmi les homopolymères résultant de la polymérisation d'un monomère à chaîne cristallisable choisi parmi les acrylates d'alkyle saturés en C₁₄-C₂₂ et les méthacrylates d'alkyle saturés en C₁₄-C₂₂, et les copolymères obtenus par copolymérisation d'un monomère à chaîne cristallisable choisi parmi les acrylates d'alkyle saturés en C₁₄-C₂₂ et les méthacrylates d'alkyle saturés en C₁₄-C₂₂, avec l'acide acrylique, en particulier les copolymères obtenus par copolymérisation de l'acrylate de béhényle et de l'acide acrylique, ou les copolymères obtenus par copolymérisation de l'acrylate de stéaryle et de l'acide acrylique.

Comme homopolymères résultant de la polymérisation d'un monomère à chaîne cristallisable choisi parmi les acrylates d'alkyle saturés en C₁₄-C₂₂ et les méthacrylates d'alkyle saturés en C₁₄-C₂₂, on peut citer notamment ceux commercialisés sous les dénominations Intelimer® par la société Landec, décrits dans la brochure "Intelimer® polymers", Landec IP22 (Rev. 4-97). Ces polymères sont sous forme solide à température ambiante. Ils portent des chaînes latérales cristallisables et correspondent à des homopolymères d'acrylates ou méthacrylates d'alkyle en C₁₄-C₂₄ saturé. On peut citer plus particulièrement l'homopolymère d'acrylate de stéaryle (Intelimer IPA-13.1) (nom INCl: Poly C10-30 alkyl acrylate), l'homopolymère d'acrylate de béhényle (Intelimer IPA-13.6) (nom INCI : Poly C10-30 alkyl acrylate), et leurs mélanges.

La quantité de polymère(s) semi-cristallin(s) dans la composition de l'invention peut aller par exemple de 0,1 à 20 % en poids, de préférence de 0,1 à 10 % en poids et mieux de 0,5 à 5 % en poids par rapport au poids total de la composition.

### Phase huileuse

La phase huileuse de la composition contient une ou plusieurs huiles, notamment des huiles cosmétiques. La quantité de phase huileuse peut aller par exemple de 2 à 70 % en poids, de préférence de 5 à 50 % en poids, mieux de 10 à 40 % en poids par rapport au poids total de la composition.

On entend par "huile" un corps gras liquide à la température ambiante (25°C).

Comme huiles utilisables dans la composition de l'invention, on peut citer par exemple :
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles d'amande douce, de tournesol, de maïs, de soja, de courge, de coriandre, de pépins de raisin, de sésame, de noisette, d'abricot (Prunus Armenica Oil), de macadamia, d'arara, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité ;
- les esters et les éthers de synthèse, notamment d'acides gras, comme les huiles de formules R¹COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin ou 660084 PCL-LIQUID de la société Symrise (mélange d'éthyl-2 hexanoate de cétylstéaryle et de myristate d'isopropyle), l'isononanoate d'isononyle, le myristate d'isopropyle, le palmitate d'isopropyle, le palmitate d'éthyl-2-hexyle (ou palmitate d'octyle), le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de Parléam® ;
- les alcools gras ayant de 8 à 26 atomes de carbone, comme l'alcool cétylique, l'alcool stéarylique et leur mélange (alcool cétéarylique), l'octyldodécanol, le 2-butyloctanol, le 2-hexyldécanol, le 2-undécylpentadécanol, l'alcool oléique ou l'alcool linoléique ;
- les huiles fluorées partiellement hydrocarbonées et/ou siliconées comme celles décrites dans le document JP-A-2-295912. Comme huiles fluorées, on peut citer aussi le perfluorométhylcyclopentane et le perfluoro-1,3 diméthylcyclohexane, vendus sous les dénominations de "FLUTEC PC1^{®}" et "FLUTEC PC3^{®}" par la Société BNFL Fluorochemicals ; le perfluoro-1,2-diméthylcyclobutane ; les perfluoroalcanes tels que le dodécafluoropentane et le tétradécafluorohexane, vendus sous les dénominations de "PF 5050^{®}" et "PF 5060^{®}" par la Société 3M, ou encore le bromoperfluorooctyle vendu sous la dénomination "FORALKYL^{®}" par la Société Atochem ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthyl-phénylsiloxanes ;
- leurs mélanges.

On entend par "huile hydrocarbonée" dans la liste des huiles citées ci-dessus, toute huile comportant majoritairement des atomes de carbone et d'hydrogène, et éventuellement des groupements ester, éther, fluoré, acide carboxylique et/ou alcool.

### Emulsionnants

La composition peut contenir un ou plusieurs émulsionnants dont la nature dépend du sens de l'émulsion et qui permettent la stabilisation du mélange des phases aqueuse et huileuse. La quantité (en matière active) d'émulsionnant(s) va de préférence de 0,01 à 10 % en poids, mieux de 0,05 à 5 % en poids et encore mieux de 0,1 à 3 % en poids de matière active par rapport au poids total de la composition.

Ces émulsionnants peuvent être choisis parmi les émulsionnants amphotères, anioniques, cationiques ou non ioniques, utilisés seuls ou en mélange. Ce sont de préférence des émulsionnants non ioniques. Ces émulsionnants sont choisis de manière appropriée suivant la phase continue de l'émulsion à obtenir (E/H ou H/E). Quand l'émulsion est multiple, elle comporte généralement un émulsionnant dans l'émulsion primaire et un émulsionnant dans la phase externe dans laquelle est introduite l'émulsion primaire.

Comme émulsionnants utilisables pour la préparation des émulsions E/H, on peut citer par exemple les alkyl esters ou alkyl éthers de sorbitan, de glycérol ou de sucres ; les tensioactifs siliconés comme les dimethicone copolyols tels que le mélange de cyclomethicone et de dimethicone copolyol, vendu sous les dénominations DC5225C et DC3225C par la société Dow Corning, et comme les alkyl-dimethicone copolyols tels que le Laurylmethicone copolyol vendu sous la dénomination "Dow Corning 5200 Formulation Aid" par la société Dow Corning, le Cetyl dimethicone copolyol vendu sous la dénomination Abil EM 90® par la société Goldschmidt-Degussa (nom INCl : Cetyl PEG/PPG-10/1 dimethicone), et le mélange de Polyglyceryl-4 isostearate/Cetyl dimethicone copolyol/Hexyl laurate vendu sous la dénomination Abil WE 09® par la société Goldschmidt-Degussa (nom INCI : Polyglyceryl-4 Isostearate / hexyl laurate / Cetyl PEG/PPG-10/1 dimethicone). On peut y ajouter aussi un ou plusieurs co-émulsionnants, qui, de manière avantageuse, peuvent être choisis dans le groupe comprenant les esters d'acide gras à chaîne ramifiée et de polyol, et notamment les esters d'acide gras à chaîne ramifiée et de glycérol et/ou de sorbitan et par exemple l'isostéarate de polyglycéryle, tel que le produit commercialisé sous la dénomination Isolan Gl34® par la société Goldschmidt-Degussa, l'isostéarate de sorbitan, tel que le produit commercialisé sous la dénomination Arlacel 987® par la société Uniqema, l'isostéarate de sorbitan et de glycérol, tel que le produit commercialisé sous la dénomination Arlacel 986® par la société Uniqema, et leurs mélanges. Ces co-émulsionnants peuvent être directement en mélange avec l'émulsionnant comme c'est le cas pour l'Abil WE 09.

Comme émulsionnants utilisables pour la préparation des émulsions H/E, on peut citer par exemple les émulsionnants non ioniques tels que les esters d'acides gras et de polyols oxyalkylénés (plus particulièrement polyoxyéthylénés), et par exemple les stéarates de polyéthylène glycol comme le stéarate de PEG-100, le stéarate de PEG-50 et le stéarate de PEG-40 ; et les mélanges les contenant tels que le mélange de stéarate de glycéryle et de stéarate de polyéthylène glycol (100 OE) (nom INCI : Glyceryl Stearate / PEG-100 Stearate) commercialisé sous la dénomination SIMULSOL 165® par la société SEPPIC ou sous la dénomination Arlacel 165® par la société Uniqema ; les esters d'acides gras et de sorbitan oxyalkylénés comprenant par exemple de 20 à 100 OE, et par exemple ceux commercialisés sous les dénominations commerciales Tween 20® ou Tween 60® par la société Ubiqema ; les éthers d'alcools gras oxyalkylénés (oxyéthylénés et/ou oxypropylénés) ; les esters de sucres comme le stéarate de sucrose ; et leurs mélanges.

On peut ajouter à ces émulsionnants, des co-émulsionnants tels que par exemple les alcools gras ayant de 8 à 26 atomes de carbone, comme l'alcool cétylique, l'alcool stéarylique et leur mélange (alcool cétéarylique), l'octyl dodécanol, le 2-butyloctanol, le 2-hexyldécanol, le 2-undécylpentadécanol ou l'alcool oléique.

On peut aussi préparer des émulsions sans émulsionnants ou en contenant moins de 0,5 % du poids total de la composition, en utilisant des composés appropriés, par exemple les polymères ayant des propriétés émulsionnantes tels que les polymères commercialisés sous les dénominations Carbopol 1342 et Pemulen par la société Noveon ; ou les polymères et copolymères d'acide 2-acrylamido 2-méthylpropane sulfonique (AMPS). On peut aussi préparer des émulsions sans émulsionnants, stabilisées par des particules siliconées ou des particules d'oxyde métallique tels que TiO2 ou autres.

### Adjuvants

La composition utilisée pour imprégner le substrat peut comprendre en outre les adjuvants classiquement mis en oeuvre dans le domaine cosmétique ou dermatologique. Elle peut contenir en particulier un ou plusieurs adjuvants choisis parmi les tensioactifs moussants, les solvants organiques, les adoucissants, les antioxydants, les chélateurs, les parfums, les filtres U.V., les matières colorantes, les actifs hydrophiles ou lipophiles, les gélifiants hydrophiles, les conservateurs, les vésicules lipidiques qui peuvent encapsuler éventuellement un ou plusieurs actifs, ou tout autre ingrédient habituellement utilisé en cosmétique ou dermatologie, et leurs mélanges. Les quantités de ces différents adjuvants sont celles classiquement utilisées dans les domaines considérés. Bien entendu, ces adjuvants doivent être de nature et utilisés en quantité telles qu'ils ne perturbent pas la composition selon l'invention. La quantité de ces adjuvants peut aller par exemple de 0,01 à 30 % en poids par rapport au poids total de la composition.

Comme tensioactifs moussants pouvant être contenus dans la composition selon l'invention, notamment pour les articles, en particulier lingettes, de nettoyage ou de démaquillage de la peau, on peut citer par exemple :
(1) parmi les tensioactifs non ioniques, les polymères blocs oxyéthylénés oxypropylénés tels que le Poloxamer 184 (nom INCl) ; les alkylpolyglycosides et notamment les alkylpolyglucosides (APG) ayant un groupe alkyle comportant de 6 à 30 atomes de carbone (alkyl-C₆-C₃₀ polyglucosides) et de préférence 8 à 16 atomes de carbone, comme par exemple le decylglucoside (Alkyl-C9/C11-polyglucoside(1.4)) tel que le produit commercialisé sous la dénomination MYDOL 10 par la société Kao Chemicals, le produit commercialisé sous la dénomination PLANTAREN 2000 UP ou PLANTACARE 2000 UP par la société Cognis, et le produit commercialisé sous la dénomination ORAMIX NS 10 par la société Seppic ; le caprylyl/capryl glucoside comme le produit commercialisé sous la dénomination ORAMIX CG 110 par la Société Seppic ; le laurylglucoside comme les produits commercialisés sous les dénominations PLANTAREN 1200 N et PLANTACARE 1200 par la société Cognis ; et le coco-glucoside comme le produit commercialisé sous la dénomination PLANTACARE 818/UP par la société Cognis ;
(2) parmi les tensioactifs anioniques, les alkylsulfates, les alkyl éther sulfates et leurs sels, notamment leurs sels de sodium, comme le mélange de Sodium Laureth Sulfate / Magnesium Laureth Sulfate / Sodium Laureth-8 Sulfate / Magnesium Laureth-8 Sulfate, vendu sous le nom de Texapon ASV par la société Cognis ; le lauryl éther sulfate de sodium (C12-14 70/30) (2,2 OE) commercialisé sous les dénominations SIPON AOS 225 ou TEXAPON N702 PATE par la société Cognis, le lauryl éther sulfate d'ammonium (C12-14 70/30) (3 OE) commercialisé sous la dénomination SIPON LEA 370 par la société Cognis ; l'alkyl (C12-C14) éther (9 OE) sulfate d'ammonium commercialisé sous la dénomination RHODAPEX AB/20 par la société Rhodia Chimie ;
(3) parmi les tensioactifs amphotères ou zwitterioniques, les dérivés alkylamido alkylamines tels que le N-cocoyl-N-carboxyméthoxyéthyl-N-carboxyméthyléthylènediamine N-di-sodique (nom INCl: Disodium cocoampho-diacetate) commercialisé en solution aqueuse saline sous la dénomination MIRANOL C2M CONC NP par la société Rhodia Chimie ; le N-cocoyl-N-hydroxyéthyl-N-carboxyméthyléthylènediamine N-sodique (nom lNCl : sodium cocampho-acetate) et le mélange d'éthanolamides d'acide de coco (nom INCI : Cocamide DEA).

Par ailleurs, la composition imprégnée dans l'article de l'invention peut contenir un ou plusieurs gélifiants hydrophiles. Ces gélifiants peuvent être choisis notamment parmi les polymères naturels ou les polymères de synthèse. Comme polymères naturels, on peut citer les polysaccharides tels que les gommes et notamment la gomme de xanthane, et les dérivés cellulosiques tels que l'hydroxyméthylcellullose et l'hydroxypropylcellulose. Comme polymères de synthèse, on peut citer les polymères carboxyvinyliques modifiés ou non, tels que les produits commercialisés sous les dénominations Carbopol (nom lNCl : carbomer) et Pemulen (nom lNCl : Acrylates/C10-30 akyl acrylate crosspolymer) par la société Noveon ; ou tels que le polyacrylate de sodium réticulé commercialisé sous la dénomination Cosmedia SP par la société Cognis (nom lNCl : Sodium polyacrylate) ou le Norsocryl S35 vendu par la société ATOFINA ; ou les polyacrylates et polyméthacrylates tels que les produits vendus sous les dénominations de Lubrajel et Norgel par la société GUARDIAN ou sous la dénomination Hispagel par la société HISPANO CHIMICA ; les polyacrylamides ; les polymères et copolymères d'acide 2-acrylamido 2-méthylpropane sulfonique, éventuellement réticulés et/ou neutralisés, comme le poly(acide 2-acrylamido 2-méthylpropane sulfonique) commercialisé par la société CLARIANT sous la dénomination Hostacerin AMPS (nom lNCl : ammonium polyacryldimethyltauramide) ; les copolymères anioniques réticulés d'acrylamide et d'AMPS, se présentant sous la forme d'une émulsion E/H, tels ceux commercialisés sous le nom de SEPIGEL 305 (nom INCI : Polyacrylamide / C13-14 Isoparaffin / Laureth-7) et sous le nom de SIMULGEL 600 (nom INCI : Acrylamide / Sodium acryloyldimethyltaurate copolymer / Isohexadecane / Polysorbate 80) par la société SEPPIC. La quantité de gélifiant(s) hydrophile(s) doit être telle qu'elle n'entrave pas les qualités de la composition, c'est-à-dire que la composition puisse s'étaler sur le support à imprégner. Cette quantité peut aller par exemple de 0,01 à 5 % en poids, de préférence de 0,05 à 3 % en poids, et mieux de 0,05 à 1 % en poids par rapport au poids total de la composition.

La composition selon l'invention peut contenir en outre une ou plusieurs charges. Comme charges, la composition peut contenir par exemple des particules minérales telles que les argiles, les silices, les oxydes métalliques tels que le dioxyde de titane ou l'oxyde de zinc, le mica, et/ou des charges organiques telles que les particules de polyamide (Nylon) et notamment celles vendues sous les dénominations ORGASOL par la société Atochem ; les poudres de polyéthylène ; les microsphères à base de copolymères acryliques, telles que celles en copolymère diméthacrylate d'éthylène glycol/ methacrylate de lauryle vendues par la société Dow Corning sous la dénomination de POLYTRAP ; les microsphères de polyméthacrylate de méthyle, commercialisées sous la dénomination MICROSPHERE M-100 par la société Matsumoto ou sous la dénomination COVABEAD LH85 par la société Wackherr ; les poudres de copolymère éthylène-acrylate, comme celles commercialisées sous la dénomination FLOBEADS par la société Sumitomo Seika Chemicals ; les poudres expansées telles que les microsphères creuses et notamment, les microsphères formées d'un terpolymère de chlorure de vinylidène, d'acrylonitrile et de méthacrylate et commercialisées sous la dénomination EXPANCEL par la société Kemanord Plast ; les poudres de matériaux organiques naturels tels que les poudres d'amidon, notamment d'amidons de maïs, de blé ou de riz, réticulés ou non, telles que les poudres d'amidon réticulé par l'anhydride octénylsuccinate, commercialisées sous la dénomination DRY-FLO par la société National Starch ; les microbilles de résine de silicone telles que celles commercialisées sous la dénomination TOSPEARL par la société Toshiba Silicone, notamment TOSPEARL 240 ; et leurs mélanges. La quantité de charge(s) peut aller par exemple de 0,05 à 10 % en poids et mieux 0,1 à 5 % en poids par rapport au poids total de la composition.

Comme actifs utilisables dans les compositions de l'invention, on peut citer par exemple, les agents hydratants tels que les hydrolysats de protéines ; le hyaluronate de sodium ; les polyols comme la glycérine, les glycols comme les polyéthylène glycols, et les dérivés de sucre ; les anti-inflammatoires ; les oligomères procyannidoliques ; les vitamines comme la vitamine A (rétinol), la vitamine E (tocophérol), la vitamine C (acide ascorbique), la vitamine B5 (panthénol), la vitamine B3 (niacinamide), les dérivés de ces vitamines (notamment esters) et leurs mélanges ; l'urée ; la caféine ; les dépigmentants tels que l'acide kojique, l'hydroquinone et l'acide caféique ; l'acide salicylique et ses dérivés ; les alpha-hydroxyacides tels que l'acide lactique et l'acide glycolique et leurs dérivés ; les rétinoïdes tels que les caroténoïdes et les dérivés de vitamine A ; les filtres solaires ; les extraits d'algues, de champignons, de végétaux, de levures, de bactéries ; les actifs antiséborrhéiques qui permettent un nettoyage de l'excédent de sébum sur la peau ; les actifs anti-bactériens comme le 2,4,4'-trichloro-2'-hydroxy diphényl éther (ou triclosan), le 3,4,4'-trichlorocarbanilide (ou triclocarban) et les acides indiqués ci-dessus et notamment l'acide salicylique et ses dérivés ; les agents matifiants ; les agents tenseurs ; les céramides ; les huiles essentielles ; et leurs mélanges ; et tout actif approprié pour le but final de la composition.

Le ou les actifs peuvent être par exemple présents en une concentration allant de 0,01 à 20 %, de préférence de 0,1 à 5 % et mieux de 0,5 à 3 % du poids total de la composition.

Bien entendu, l'homme de l'art veillera à choisir le ou les éventuels composés à ajouter aux compositions de l'invention de manière telle que les propriétés avantageuses attachées intrinsèquement au produit conforme à l'invention ne soient pas ou substantiellement pas, altérées par l'addition envisagée.

### Substrat

Le substrat insoluble dans l'eau peut comprendre une ou plusieurs couches et il peut être choisi dans le groupe comprenant des matériaux tissés, des matériaux non-tissés, des mousses, des éponges, des ouates, en feuilles, boules ou films. Il peut s'agir notamment d'un substrat non tissé à base de fibres d'origine naturelle (lin, laine, coton, soie, fibres de bambou) ou d'origine synthétique (dérivés de cellulose, viscose, dérivés polyvinyliques, polyesters comme téréphtalate de polyéthylène, polyoléfines comme polyéthylène (PET)) ou polypropylène, polyamides comme le Nylon, dérivés acryliques), et leurs mélanges tels que viscose/PET, acide polylactique (PLA), viscose/acide polylactique (viscose/PLA).

Selon un mode préféré de réalisation de l'invention, le substrat est un non-tissé. Les non tissés sont décrits de façon générale dans RIEDEL « Nonwoven Bonding Methods & Materials », Nonwoven World (1987). Ces substrats sont obtenus selon les procédés usuels de la technique de préparation des non-tissés.

Quand le substrat est un non-tissé, on utilise de préférence un non-tissé qui ne se met pas en boule et qui est assez solide pour ne pas se déliter et ne pas pelucher à l'application sur la peau. Il doit être absorbant, doux au moins sur une face pour le démaquillage des yeux en particulier. Comme non-tissés appropriés, on peut citer par exemple ceux commercialisés sous les dénominations Ultraloft 15285-01, Ultraloft 182-008, Ultraloft 182-010, Ultraloft 182-016 par la société BBA, Vilmed M1519 Blau, Vilmed M 1550 N et 112-132-3 par la société Freudenberg, celui commercialisé sous la dénomination Norafin 11601-010B par la société Jacob Holm Industries, les non tissés flockés commercialisés sous les dénominations Univel 109 et Univel 119 par la société Uni Flockage, et celui en Viscose/PLA fourni par la société Sandler.

Ce substrat peut comporter une ou plusieurs couches ayant des propriétés identiques ou différentes et ayant des propriétés d'élasticité, de douceur et autres appropriées à l'usage recherché. Les substrats peuvent comporter par exemple deux parties ayant des propriétés d'élasticité différentes, comme décrit dans le document WO-A-99/13861 ou comporter une seule couche avec des densités différentes comme décrit dans le document WO-A-99/25318, ou encore comporter deux couches de textures différentes comme décrit dans le document WO-A-98/18441.

En outre, quand l'article est utilisé pour le corps, le substrat peut comprendre au moins une face rugueuse pour permettre en même temps le massage de la peau ou le gommage de la peau.

Comme indiqué plus haut, le substrat peut avoir toute taille et toute forme appropriées au but recherché. Par ailleurs, il a généralement une surface comprise entre 0,005 m² et 0,1 m², de préférence entre 0,01 m² et 0,05 m².

Le taux d'imprégnation de la composition sur le substrat va généralement de 100 à 1000 %, de préférence de 250 à 700 % du poids du substrat. Les techniques d'imprégnation des substrats par des compositions sont bien connues dans ce domaine et sont toutes applicables à la présente invention. En général, la composition d'imprégnation est chauffée et ajoutée au substrat par une ou plusieurs techniques comprenant l'immersion, l'enduction, la vaporisation, etc.

L'invention a aussi pour objet un procédé cosmétique de soin de la peau, de nettoyage et/ou de démaquillage de la peau, consistant à appliquer sur la peau, un article tel que défini ci-dessus.

### Exemples de composition

Les exemples ci-après de compositions selon l'invention sont donnés à titre d'illustration et sans caractère limitatif. Les noms sont en nom chimique ou en nom INCl. Les quantités y sont données en % en poids, sauf mention contraire.

### Exemple : Emulsion E/H

| Composés | Quantités en % |
|---|---|
| Phase A | |
| Cetyl PEG/PPG-10/1 dimethicone (Abil EM90) | 1,5 |
| Polyglyceryl-4 isostearate (Isolan G134) | 0,5 |
| Isohexadecane | 11 |
| Conservateurs | 0,1 |
| Poly C10-30 alkyl acrylate (Intelimer IPA-13.1 | 1,3 |
| Huile d'abricot (Prunus Armenica OII) | 5 |

| Phase B | |
|---|---|
| Cyclohexasiloxane | 9 |
| Acrylate copolymer (Cosmedia SP) | 0,5 |
| Aluminium starch octenylsuccinate (Dry-Flo) | 1,5 |

| Phase C | |
|---|---|
| Eau | 58,4 |
| Conservateurs | 0,5 |
| Glycérine | 10 |
| Sulfate de magnésium | 0,7 |

### Mode opératoire :

La phase A a été chauffée à 70°C sous agitation jusqu'à obtention d'une phase homogène, puis ramenée à 65°C.
La phase B a été agitée à température ambiante jusqu'à obtention d'une bonne dispersion des charges puis ajoutée à la phase A.
La phase C a été ensuite chauffée sous agitation à 85°C jusqu'à obtention d'une phase limpide puis la température a été ramenée à 65°C.
La phase C a été ensuite ajoutée au mélange de A + B pour la mise en émulsion sous Moritz. Le mélange a été ramené à 25 °C.

On a obtenu une émulsion E/H visqueuse, apte à imprégner un substrat en forme de lingette de manière homogène. Pour l'imprégnation du substrat, l'émulsion a été réchauffée à 60°C, ce qui l'a fluidifiée, puis elle a été dispersée sur le substrat à chaud. Le substrat était un non-tissé de type viscose/PLA en forme de lingette, le taux d'imprégnation étant de 350 %. La lingette imprégnée a été ensuite refroidie jusqu'à température ambiante (environ 20°C).

Le lingette imprégnée ainsi obtenue peut être utilisée pour le soin de la peau. Elle peut aussi être appliquée en tant que masque sur le visage.

### Exemple 2 : Emulsions E/H

| Composés | Composition selon l'invention (%) | Composition comparative (%) |
|---|---|---|
| Phase A | | |
| Cetyl PEG/PPG-10/1 dimethicone (Abil EM90) | 1,5 | 1,5 |
| Polyglyceryl-4 isostearate (Isolan G134) | 0,5 | 0,5 |
| Isohexadecane | 11 | 11 |
| Conservateurs | 0,1 | 0,1 |
| Poly C10-30 alkyl acrylate (Intelimer IPA-13.1 | **1** | - |
| Palmitate de dextrine (Rheopearl TL) | | **1** |
| Huile d'abricot (Prunus Armenica Oll) | 5 | 5 |

| Phase B | | |
|---|---|---|
| Cyclohexasiloxane | 9 | 9 |
| Acrylate copolymer (Cosmedia SP) | 0,5 | 0,5 |
| Aluminium starch octenylsuccinate (Dry-Flo) | 1,5 | 1,5 |

| Phase C | | |
|---|---|---|
| Eau | 58,4 | 58,4 |
| Conservateurs | 0,5 | 0,5 |
| Glycérine | 10 | 10 |
| Sulfate de magnésium | 0,7 | 0,7 |

### Mode opératoire :

La phase A a été chauffée à 70°C sous agitation jusqu'à obtention d'une phase homogène, puis ramenée à 40°C.
La phase B a été agitée à température ambiante jusqu'à obtention d'une bonne dispersion des charges puis ajoutée à la phase A.
La phase C a été ensuite chauffée sous agitation à 85°C jusqu'à obtention d'une phase limpide puis la température a été ramenée à 25°C.
La phase C a été ensuite ajoutée au mélange de A + B pour la mise en émulsion sous Moritz..

On a obtenu des émulsions E/H visqueuses,.
Pour l'imprégnation du substrat, chaque composition est réchauffée à 65°C, puis dispersée sur le substrat à chaud. Le substrat était un non-tissé de type viscose/PLA en forme de lingette, le taux d'imprégnation étant de 350 %. La lingette imprégnée a été ensuite refroidie jusqu'à température ambiante (environ 20 °C).
Dans le cas de la composition selon l'invention, la composition réchauffée se fluidifie et vient imprégner le substrat de manière homogène. La composition comparative, après avoir été échauffée à 65°C, reste très visqueuse et ne permet pas une imprégnation homogène du support.
L'utilisation du polymère semi-cristallin permet donc bien d'imprégner de manière correcte et homogène le substrat.

## Revendications

1. Article cosmétique comportant (A) un substrat insoluble dans l'eau, comprenant une ou plusieurs couches, et (B) une composition ajoutée ou imprégnée sur le substrat, comprenant au moins une phase aqueuse, au moins une phase huileuse, et un ou plusieurs polymères semi-cristallins.

2. Article selon la revendication 1, **caractérisé en ce que** le polymère semi-cristallin est choisi parmi les homopolymères et copolymères résultant de la polymérisation d'au moins un monomère à chaîne(s) cristallisable(s), la chaîne cristallisable étant choisie parmi les chaînes alkyles comportant au moins 11 atomes de carbone et au plus 40 atomes de carbone.

3. Article selon la revendication 1 ou 2, **caractérisé en ce que** le polymère semi-cristallin est choisi parmi les homopolymères résultant de la polymérisation d'un monomère à chaîne cristallisable choisi parmi les acrylates d'alkyle saturés en C₁₄-C₂₂ et les méthacrylates d'alkyle saturés en C₁₄-C₂₂ ; les copolymères obtenus par copolymérisation d'un monomère à chaîne cristallisable choisi parmi les acrylates d'alkyle saturés en C₁₄-C₂₂ et les méthacrylates d'alkyle saturés en C₁₄-C₂₂, avec l'acide acrylique.

4. Article selon l'une quelconque des revendications précédentes, **caractérisé en ce que** le polymère semi-cristallin est choisi parmi l'homopolymère d'acrylate de stéaryle, l'homopolymère d'acrylate de béhényle, et leurs mélanges.

5. Article selon l'une quelconque des revendications précédentes, **caractérisé en ce que** la quantité de polymère(s) semi-cristallin(s) va de 0,1 à 20 % en poids par rapport au poids total de la composition (B).

6. Article selon l'une quelconque des revendications précédentes, **caractérisé en ce que**. la quantité de phase huileuse va de 2 à 70 % en poids et de préférence de 5 à 50 % en poids par rapport au poids total de la composition (B).

7. Article selon l'une quelconque des revendications précédentes, **caractérisé en ce que** la composition (B) contient en outre au moins un émulsionnant.

8. Article selon l'une quelconque des revendications précédentes, **caractérisé en ce que** la composition (B) contient en outre un ou plusieurs gélifiants hydrophiles.

9. Article selon l'une quelconque des revendications précédentes, **caractérisé en ce que** la quantité de gélifiant(s) hydrophile(s) va de 0,01 à 5 % en poids par rapport au poids total de la composition (B).

10. Article selon l'une quelconque des revendications précédentes, **caractérisé en ce que** la composition (B) est une émulsion.

11. Article selon l'une quelconque des revendications précédentes, **caractérisé en ce que** la composition (B) présente une viscosité de 1 à 15 Pa.s.

12. Article selon l'une quelconque des revendications précédentes, **caractérisé en ce que** le substrat est choisi dans le groupe comprenant les matériaux tissés, les matériaux non-tissés, les mousses, les éponges, les ouates.

13. Article selon l'une quelconque des revendications précédentes, **caractérisé en ce que** le substrat est un non tissé à base de fibres d'origine naturelle et/ou d'origine synthétique.

14. Article selon l'une quelconque des revendications précédentes, **caractérisé en ce qu'**il comprend de 100 à 1000 % en poids de composition (B) par rapport au poids de substrat.

15. Article selon l'une quelconque des revendications précédentes, **caractérisé en ce qu'**il constitue un article de soin de la peau et/ou des cheveux et/ou de nettoyage et/ou de démaquillage de la peau.

16. Utilisation cosmétique de l'article selon l'une quelconque des revendications 1 à 14, pour le soin de la peau ou des cheveux, et/ou le nettoyage et/ou le démaquillage et/ou le gommage de la peau.
